# EUROPEAN PATENT SPECIFICATION

(11) **EP 2 452 629 B1**
(45) Date of publication and mention of the grant of the patent: **02.03.2016**
(21) Application number: 11188365.8
(22) Date of filing: 09.11.2011
(51) Int. Cl.: A61B 17/00, A61B 90/30

(54) **Surgical instrument with add-on power adapter for accessory**
OP-Instrument mit zusätzlichem Stromadapter als Zubehör
Instrument chirurgical doté d'un adaptateur de puissance complémentaire en tant qu'accessoire

(30) Priority: 10.11.2010 US 412115 P; 26.10.2011 US 201113281569
(43) Date of publication of application: 16.05.2012
(73) Proprietor: Covidien LP, Mansfield, MA 02048 (US)
(72) Inventor: Viola, Frank, Sandy Hook, Connecticut 06482 (US)
(74) Representative: Soames, Candida Jane

(56) References cited:
- EP-A1- 1 913 864
- WO-A2-2004/035106
- WO-A2-2009/134634
- US-A- 4 542 741
- US-A- 5 785 408
- US-A- 5 951 142
- US-A- 6 029 303
- US-A1- 2006 291 195

## Description

### BACKGROUND

This application relates to surgical instruments and more particularly, to an adapter for surgical instruments that provides power to accessories for use with surgical instruments.

### Background of Related Art

A typical surgery employs a plurality of different surgical instruments and accessory devices for use with the various surgical instruments. When attaching accessory devices, e.g. illumination devices or cameras, there is often a need to satisfy the energy needs of the accessory device. While self contained energy sources like batteries are often utilized, they take up valuable space in the accessory device, thereby increasing the size of the accessory which is disadvantageous in minimally invasive surgery. Also, such energy sources often have limited energy storage capacity. As such, removal or repositioning of the accessory may be necessary to change a battery or other energy storage device, which, if required during surgery or other medical procedure, can increase the time and complexity of the surgery and reduce efficiency. Examples of such prior art devices include EP1913864A1 (Medic NRG Ltd), US2006/291195 (Horrell Robin, et al.,), WO2004/035106 (Given Imaging Ltd), WO2009/134634 (Vivid Medical Inc et al.), which is considered as the closest prior art document and which discloses a surgical instrument system according to the preamble of claim 1, US5951142 (Wang Scott Chien-Kuo et al.,), US4542741 (Burgin Kermit) and US5785408 (Tseng Kuo Hwa).

### SUMMARY

Accordingly, the present disclosure in one aspect is directed to a surgical instrument system that includes a surgical instrument, an accessory, and an adapter. The surgical instrument includes a housing, a shaft extending from the housing, and a tool assembly operably coupled to the shaft. The accessory is operably couplable to the surgical instrument. The adapter is operably coupled to the surgical instrument and includes an electrical energy storage device to provide power to the accessory when the accessory is operably coupled to the surgical instrument, wherein the adapter is electrically coupled to the surgical instrument via a slip ring arrangement comprising a ring adapted to be positioned on the shaft and adapted to engage an inner annular surface of the adapter to facilitate the transmission of electrical power therethrough in order to transmit the electrical power from the adapter to the accessory.

The energy storage device may be a battery. The accessory may include one or more of a camera and an illumination device.

In one embodiment, the surgical instrument includes a base portion disposed adjacent one or more of the shaft and the housing. The base portion can be configured and dimensioned to engage the adapter such that the adapter transmits power to the base portion and the base portion transmits power to the accessory. The base portion may include one or more contacts.

The adapter may include one or more contacts. The one or more contacts of the adapter and the one or more contacts of the base portion can be configured and dimensioned to engage one another to transmit the power from the adapter to the base portion. The adapter can define an energy storage housing for positioning a battery therein. The adapter can be slidably coupled onto the shaft.

The accessory can include at least one contact to engage a contact of the instrument. The contact for the accessory can be distal of the contact for the adapter.

In another aspect, the present disclosure provides a surgical instrument system for powering an accessory comprising a shaft supporting at least one contact and an adapter operably coupled to the shaft. The adapter includes an energy storage device to provide power to the accessory when the accessory is operably coupled to the surgical instrument in electrical connection with the at least one contact.

In some embodiments, the surgical instrument includes a base portion disposed adjacent to at least one of the shaft and the housing, and the adapter engages the base portion of the surgical instrument such that the adapter transmits power to the base portion and the base portion transmits power to the accessory.

In some embodiments, the adapter includes at least one contact and the surgical instrument includes at least one contact, wherein the contacts are configured and dimensioned to engage one another to transmit the power from the adapter to the surgical instrument for transmission to the accessory.

### BRIEF DESCRIPTION OF THE DRAWINGS

Various embodiments of the present disclosure are described herein with reference to the drawings wherein:
Fig. 1 is a perspective view of one embodiment of a surgical instrument system in accordance with the present disclosure;
Fig. 1A is a perspective view of the accessory and distal portion of the instrument of Figure 1;
Fig. 2 is a partial perspective view, with parts separated, illustrating the positioning of one embodiment of an adapter on the surgical instrument system of Fig. 1;
Fig. 3 is a bottom perspective view of the adapter illustrated in Fig. 2;
Fig. 4 is a partial perspective view, with parts separated, illustrating the positioning of another embodiment of an adapter on another embodiment of a surgical instrument;
Fig. 5 is a bottom perspective view of the adapter illustrated in Fig. 4; and
Fig. 6 is a perspective of another instrument of the present disclosure.

### DETAILED DESCRIPTION

Embodiments of the presently disclosed surgical instrument are described in detail with reference to the drawings, wherein like reference numerals designate similar or identical elements in each of the several views. In the drawings and the description that follows, the term "proximal" refers to the end of the surgical instrument that is closer to the user, whereas the term "distal" refers to the end of the surgical instrument that is further from the user.

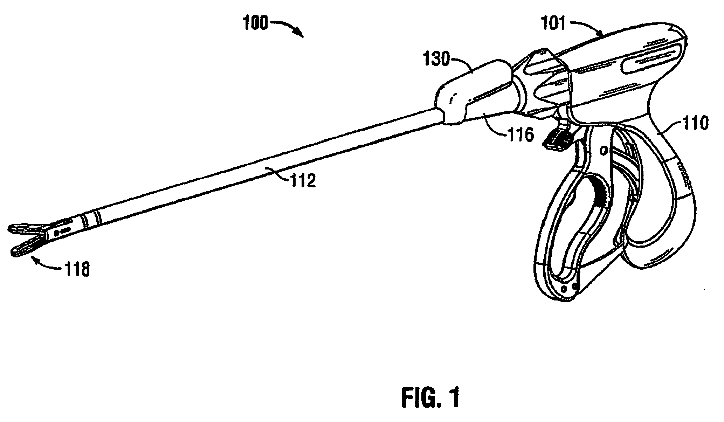

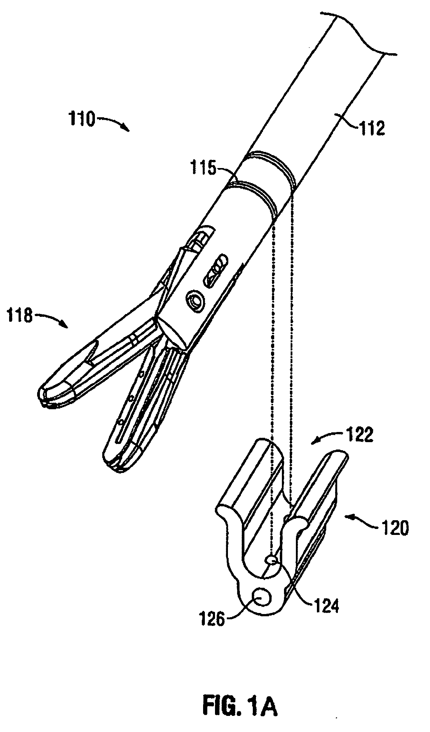

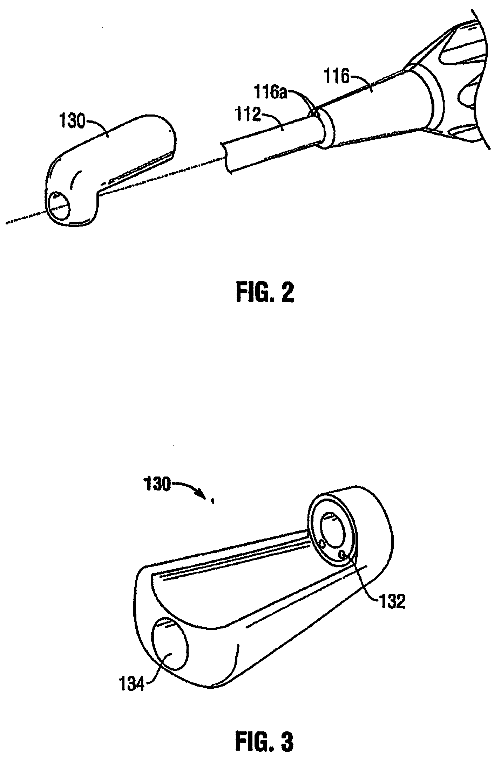

Referring now to Figs. 1 and 2, one embodiment of a surgical instrument system 100 is illustrated. The surgical instrument system 100 includes a surgical instrument 101, an adapter 130, and an accessory 120. The instrument illustrated is an endoscopic grasper, by way of example, it being understood that other instruments can also be utilized such as endoscopic scissors, clip appliers, surgical staplers, etc. The surgical instrument 101 has a housing 110, an elongated shaft 112, a base portion 116, and a tool assembly 118. The shaft 112 extends from the housing 110 and base portion 116. The tool assembly 118 is disposed on the distal end portion of the shaft 112. The base portion 116 is disposed at a proximal end portion of shaft 112.

The surgical instrument 110 includes one or more contacts 115. In the illustrated embodiment, two ring contacts 115 are shown by way of example. The contacts 115 may be positioned on the shaft 112. The contacts 115 may include contact buttons, slip rings, or any other suitable connection. The contacts 115 are electrically coupled to the adapter 130, which contains an energy storage device, e.g., via one or more wires extending therebetween (through the shaft 112) and/or a wireless connection such that the energy storage device transmits power to the contacts 115. The contacts 115 are engaged by the contacts 124 of the accessory 120. In this manner, the power is transmitted to the accessory 120 from the adapter 130 through the contacts 115, 124.

Referring now to Fig. 1A, the accessory 120 is mountable, and preferably removably mountable, to the surgical instrument 110. The accessory 120 defines a channel 122 therethrough and includes one or more contacts 124. The channel 122 is configured and dimensioned to accommodate at least a portion of the shaft 112 when the accessory 120 is selectively operably coupled to the surgical instrument 110. Thus, the accessory 120 can frictionally engage the shaft 112. Alternatively, the accessory can be attached to the shaft by a snap fit, interlocking structure or by other methods. The contacts 124 may be positioned within the channel 122 such that when the accessory 120 is positioned on the shaft 112 adjacent contacts 115, two or more contacts (i.e., at least one contact 115 and at least one contact 124) are engaged so that the accessory 120 is electrically coupled to the energy device of adapter 130. In particular, the contacts 124 may be operably coupled to the energy device via any suitable electrical or electromechanical features. For example, these features, which may include wired or wireless connections, may have inductive components, capacitive components, resistive components, switching components, etc. that facilitate the connection between the accessory 120 and the surgical instrument 110 so that the accessory 120 can be powered by the energy device of adapter 130. Furthermore, the contacts 124 may include contact buttons, slip rings, or any other suitable connection components.

As illustrated in Fig. 2, the adapter 130 may be selectively operably coupled to the shaft 112 and/or the base portion 116 of the surgical instrument 100. In alternate embodiments, the adapter 130 is configured and dimensioned to couple to the housing of the surgical instrument. The adapter in some embodiments can be removably coupled to the instrument. As discussed above, the adapter 130 contains an energy storage device (e.g., a battery or any other suitable self-contained electrical energy source) that provides power/energy to the accessory 120 (e.g., via a wired and/or wireless connection).

It should be appreciated that other energy sources for powering the accessory 120 may also be stored in the adapter 130. The accessory 120 may be operably coupled to one or both of the adapter 130 and the surgical instrument 100 (e.g., the shaft 112, the housing 110, and/or the base portion 116). The accessory 120 may include one or more powered devices including cameras, illumination devices, or any other suitable powered device that could assist the clinician in performing a medical procedure.

The base portion 116 is configured and dimensioned to receive and engage the adapter 130 such that the adapter 130 transmits power to the base portion 116 for providing power to the accessory 120. The base portion 116 is operably coupled to the accessory 120. In this manner, the base portion 116 transmits power from the energy storage device, through the adapter 130, and to the accessory 120.

As illustrated in Figs. 2 and 3, the base portion 116 includes one or more contact buttons 116a and the adapter 130 includes one or more contact buttons 132. The one or more contact buttons 132 of the adapter 130 and the one or more contact buttons 116a of the base portion 116 are configured and dimensioned to engage one another to transmit the power from the energy storage device to the accessory 120. The adapter 130 slidably couples onto the shaft 112 such that the contact buttons 132, 116a engage one another. The adapter 130 can attach to the shaft 112 by a frictional fit, snap fit or by other methods. The adapter 130 defines an energy storage housing 134 for positioning the energy storage device therein. The energy storage housing 134 facilitates the transmission of power from the energy storage device through the contact buttons 116a, 132 by enabling the energy storage device to be positioned in electrical communication with the contact buttons 116a, 132. In this respect, the energy storage device may be in direct contact with one or more of the contact buttons 116a, 132 when positioned in the energy storage housing 134. Note in the illustrated embodiments, contact buttons 116a are positioned proximally of the more distal contacts 115.

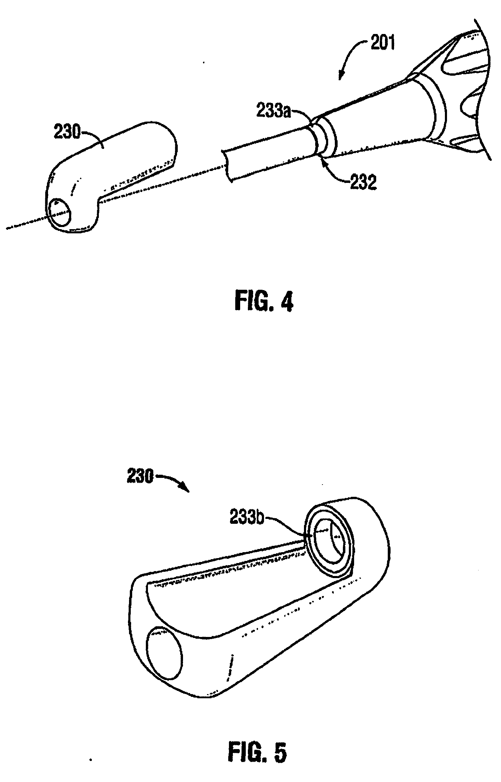

Referring now to Figs. 4 and 5, another embodiment of a surgical instrument 201 and adapter 230 are illustrated. In this embodiment, the adapter 230 is operably coupled to the surgical instrument 201 via a slip ring arrangement 232 in order to transmit power from the energy storage device to the accessory 120. In this manner, a ring 233a may be positioned on the shaft 112 that engages an inner annular surface 233b of the adapter 230 to facilitate the transmission of power therethrough to provide power to the accessory 120.

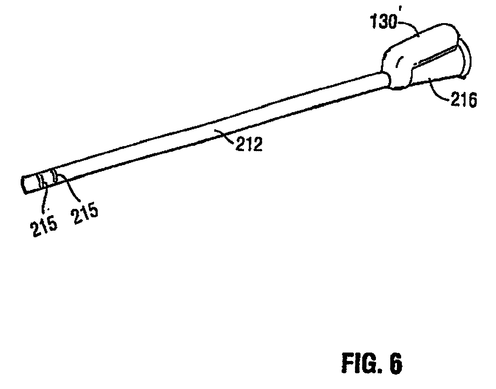

In the embodiment of Figure 6, adapter 130' can be attached to rod 212 adjacent base portion 216. An accessory, such as accessory 120 of Figure 2, can be mounted to engage the contacts 215 positioned on rod 212. Contacts 215 can be wired to the energy storage device in adapter 130' in the same manner as in Figure 1 to supply energy to the accessory 120 when mounted to the rod 212. In this manner a simple device for inserting an accessory, e.g. a camera, into a body cavity is provided.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. A surgical instrument system (100) for powering an accessory (120) comprising:
a surgical instrument (101) including a housing (110);
a shaft (112) extending from the housing;
a tool assembly (118) operably coupled to the shaft; and
an adapter (230) operably coupled to the surgical instrument, the adapter including an electrical energy storage device to provide electrical power to the accessory when the accessory is operably coupled to the surgical instrument, **characterised in that** the adapter is electrically operably coupled to the surgical instrument via a slip ring arrangement (232) comprising a ring (233a) adapted to be positioned on the shaft (112) and adapted to engage an inner annular surface (233b) of the adapter (230) to facilitate the transmission of electrical power therethrough in order to transmit the electrical power from the adapter (230) to the accessory (120).

2. The surgical instrument system according to claim 1, further comprising an accessory operably couplable to the surgical instrument.

3. The surgical instrument system of claim 2, wherein the instrument includes at least one contact adapted to be electrically connect the accessory to the surgical instrument.

4. The surgical instrument system according to claim 3, wherein at least one contact is on the shaft of the instrument.

5. The surgical instrument system according to any of claims 1-4, wherein the surgical instrument includes a base portion disposed adjacent to at least one of the shaft and the housing, the adapter being adapted to engage the base portion when coupled to the surgical instrument such that the adapter transmits power to the base portion, and the base portion transmits power to the accessory.

6. The surgical instrument system according to claim 1, wherein the energy storage device is a battery.

7. The surgical instrument system according to claim 6, wherein the adapter defines an energy storage housing for positioning the battery therein.

8. The surgical instrument system according to any of claims 1-7, wherein the adapter is slidably coupled to the shaft.

9. The surgical instrument system according to any of claims 1-8, wherein the accessory is at least one of a camera and an illumination device.

10. The surgical instrument system according to any of claims 1-9, wherein the accessory frictionally engages the shaft.

11. The surgical instrument system according to any of claims 1-10, wherein the accessory includes at least one contact to engage a distal contact of the surgical instrument.

12. The surgical instrument system according to claim 11, wherein the distal contact is positioned on the shaft.

## Patentansprüche

1. Ein chirurgisches Instrumentensystem (100) zum Versorgen eines Zubehörs (120) mit Energie, aufweisend:
ein chirurgisches Instrument (101), das ein Gehäuse (110) umfasst;
einen Schaft (112), der sich aus dem Gehäuse erstreckt;
eine Werkzeuganordnung (118), die betriebsmäßig mit dem Schaft gekuppelt ist; und
einen Adapter (230), der betriebsmäßig mit dem chirurgischen Instrument gekuppelt ist, wobei der Adapter eine elektrische Energiespeichervorrichtung umfasst, um elektrische Energie an das Zubehör zu liefern, wenn das Zubehör betriebsmäßig mit dem chirurgischen Instrument gekuppelt ist, **dadurch gekennzeichnet, dass** der Adapter elektrisch betriebsmäßig mit dem chirurgischen Instrument über eine Schleifringanordnung (232), welche einen Ring (233a) aufweist, der angepasst ist, um auf dem Schaft (112) positioniert zu werden, und angepasst ist, um mit einer inneren ringförmigen Oberfläche (233b) von dem Adapter (230) in Eingriff zu kommen, gekuppelt ist, um die Übertragung von elektrischer Energie dahindurch zu ermöglichen, um die elektrische Energie von dem Adapter (230) zu dem Zubehör (120) zu übertragen.

2. Das chirurgische Instrumentensystem gemäß Anspruch 1, weiter aufweisend ein Zubehör, das betriebsmäßig mit dem chirurgischen Instrument verbindbar ist.

3. Das chirurgische Instrumentensystem von Anspruch 2, wobei das Instrument wenigstens einen Kontakt umfasst, der angepasst ist, um das Zubehör elektrisch mit dem chirurgischen Instrument zu verbinden.

4. Das chirurgische Instrumentensystem gemäß Anspruch 3, wobei wenigstens ein Kontakt auf dem Schaft von dem Instrument ist.

5. Das chirurgische Instrumentensystem gemäß einem der Ansprüche 1 - 4, wobei das chirurgische Instrument einen Basisabschnitt umfasst, der benachbart zu wenigstens einem von dem Schaft und dem Gehäuse angeordnet ist, wobei der Adapter angepasst ist mit dem Basisabschnitt in Eingriff zu kommen, wenn mit dem chirurgischen Instrument gekuppelt, so dass der Adapter Energie zu dem Basisabschnitt liefert und der Basisabschnitt Energie zu dem Zubehör liefert.

6. Das chirurgische Instrumentensystem gemäß Anspruch 1, wobei die Energiespeichervorrichtung eine Batterie ist.

7. Das chirurgische Instrumentensystem gemäß Anspruch 6, wobei der Adapter ein Energiespeichergehäuse zum darin Positionieren der Batterie definiert.

8. Das chirurgische Instrumentensystem gemäß einem der Ansprüche 1 - 7, wobei der Adapter verschiebbar mit dem Schaft gekuppelt ist.

9. Das chirurgische Instrumentensystem gemäß einem der Ansprüche 1 - 8, wobei das Zubehör wenigstens eines von einer Kamera und einer Beleuchtungsvorrichtung ist.

10. Das chirurgische Instrumentensystem gemäß einem der Ansprüche 1 - 9, wobei das Zubehör reibschlüssig mit dem Schaft in Eingriff kommt.

11. Das chirurgische Instrumentensystem gemäß einem er Ansprüche 1 - 10, wobei das Zubehör wenigstens einen Kontakt umfasst, um mit einem distalen Kontakt von dem chirurgischen Instrument in Eingriff zu kommen.

12. Das chirurgische Instrumentensystem gemäß Anspruch 11, wobei der distale Kontakt auf dem Schaft positioniert ist.

## Revendications

1. Système d'instrument chirurgical (100) pour alimenter un accessoire (120) comprenant :
un instrument chirurgical (101) comprenant un boîtier (110) ;
un arbre (112) s'étendant du boîtier ;
un ensemble d'outil (118) couplé en service à l'arbre ; et
un adaptateur (230) couplé en service à l'instrument chirurgical, l'adaptateur comprenant un dispositif de stockage d'énergie électrique pour fournir de l'énergie électrique à l'accessoire lorsque l'accessoire est couplé en service à l'instrument chirurgical, **caractérisé en ce que** l'adaptateur est couplé électriquement en service à l'instrument chirurgical via un aménagement à contact tournant (232) comprenant un anneau (233a) adapté pour être positionné sur l'arbre (112) et qui est à même de s'engager sur la surface annulaire interne (233b) de l'adaptateur (230) pour faciliter la transmission de l'énergie électrique à travers celui-ci afin de transmettre l'énergie électrique de l'adaptateur (230) à l'accessoire (120) .

2. Système d'instrument chirurgical selon la revendication 1, comprenant en outre un accessoire qui peut être couplé en service à l'instrument chirurgical.

3. Système d'instrument chirurgical selon la revendication 2, dans lequel l'instrument comprend au moins un contact qui est à même de connecter électriquement l'accessoire à l'instrument chirurgical.

4. Système d'instrument chirurgical selon la revendication 3, dans lequel au moins un contact se trouve sur l'arbre de l'instrument.

5. Système d'instrument chirurgical selon l'une quelconque des revendications 1 à 4, dans lequel l'instrument chirurgical comprend une partie de base disposée adjacente à au moins l'un ou l'autre de l'arbre et du boîtier, l'adaptateur étant à même de s'engager sur la partie de base lorsqu'il est couplé à l'instrument chirurgical de sorte que l'adaptateur transmettre de l'énergie à la partie de base et que la partie de base transmette de l'énergie à l'accessoire.

6. Système d'instrument chirurgical selon la revendication 1, dans lequel le dispositif de stockage d'énergie est une pile.

7. Système d'instrument chirurgical selon la revendication 6, dans lequel l'adaptateur définit un boîtier de stockage d'énergie pour y positionner la pile.

8. Système d'instrument chirurgical selon l'une quelconque des revendications 1 à 7, dans lequel l'adaptateur est couplé à coulissement à l'arbre.

9. Système d'instrument chirurgical selon l'une quelconque des revendications 1 à 8, dans lequel l'accessoire est au moins l'un ou l'autre d'une caméra et d'un dispositif d'éclairage.

10. Système d'instrument chirurgical selon l'une quelconque des revendications 1 à 9, dans lequel l'accessoire s'engage à friction sur l'arbre.

11. Système d'instrument chirurgical selon l'une quelconque des revendications 1 à 10, dans lequel l'accessoire comprend au moins un contact pour s'engager sur un contact distal de l'instrument chirurgical.

12. Système d'instrument chirurgical selon la revendication 11, dans lequel le contact distal est positionné sur l'arbre.
